# EUROPÄISCHE PATENTSCHRIFT

(11) **EP 0 869 811 B1**
(45) Veröffentlichungstag und Bekanntmachung des Hinweises auf die Patenterteilung: **11.08.2004**
(21) Anmeldenummer: 96944599.8
(22) Anmeldetag: 19.12.1996
(51) Int. Cl.: A61K 38/16

(54) **IMMUNOLOGISCH WIRKSAME MISTELEXTRAKTZUBEREITUNGEN**
IMMUNOLOGICALLY ACTIVE MISTLETOE EXTRACT PREPARATIONS
PREPARATIONS D'EXTRAIT DE GUI A ACTION IMMUNOLOGIQUE

(30) Priorität: 27.12.1995 DE 19548367
(43) Veröffentlichungstag der Anmeldung: 14.10.1998
(73) Patentinhaber: MADAUS AG, 51067 Köln (DE)
(72) Erfinder: WÄCHTER, Wilfried, D-51469 Bergisch Gladbach (DE); WITTHOHN, Klaus, D-51491 Overath (DE)
(74) Vertreter: Wolff, Felix, Dr.
(86) Internationale Anmeldenummer: PCT/EP1996/005718
(87) Internationale Veröffentlichungsnummer: WO 1997/024136

(56) Entgegenhaltungen:
- EP-A- 0 602 686
- DE-A- 4 221 836
- DE-A- 4 341 476

## Beschreibung

Die Erfindung bezieht sich auf Arzneimittelzubereitungen für die parenterale Verabreichung von Mistellektin und sie umfaßt in weiterer Ausgestaltung pharmazeutische Zubereitungen aus Mistelextrakten, die sich dadurch auszeichnen, daß sie zur Vermeidung von Nebenwirkungen einen möglichst geringen immunologisch aktiven Gehalt an Mistellektin und durch stabilisierende Zusätze eine hohe Laufzeitstabilität besitzen.

Die Mistel (Viscum album) ist als Arzneipflanze seit langem bekannt. Mistelextrakte finden in Präparaten zur Behandlung rheumatischer Erkrankungen und Arthrosen Verwendung. Wäßrige Mistelzubereitungen sind als Injektionslösungen in der Palliativtherapie bei malignen Tumoren in Gebrauch. Die krebshemmenden Eigenschaften von Mistelextrakten sind vielfach untersucht und beschrieben worden. Als wirksame Inhaltsstoffe wurden die Mistellektine erkannt. [Hajto, T., Hostanska, K , Gabius, W-J , Cancer Res. 49 (1989), 4803-4808, (1); Beuth, J., Ko, H.L., Tungal, L., Geisel, J., Pulverer, G., Arzneim.-Forsch. 43 (1993), 166-169, (2)].

Mistellektine haben sowohl zytotoxische wie auch immunmodulierende Eigenschaften, so daß sie bei geeigneter Dosierung in der Lage sind, verschiedene Parameter des Immunsystems positiv zu beeinflussen. Sie gehören zur Substanzklasse der Glykoproteine und bestehen aus einer leichteren Untereinheit (A-Kette) mit ribosomeninaktivierenden Eigenschaften (RIP) und einer schwereren Untereinheit (B-Kette), die sich durch spezifische zuckerbindende Aktivität auszeichnet. Die Untereinheiten sind über Disulfid-Brücken miteinander verknüpft (Luther, P , Becker, H., Die Mistel, Berlin, Springer-Verlag 1987, 58-119, (4)).

Die hohe Toxizität von Mistelextrakten und ihren Inhaltsstoffen verlangt für die therapeutische Anwendung eine exakte Dosierung, um die Verträglichkeit von Mistelpräparaten sicherzustellen und um zu verhindern, daß durch Überdosierung immunsupprimierende Effekte ausgelöst werden. Die optimale Dosierung wird bisher mit 1 ng Mistellektin/kg Körpergewicht angegeben [Hajto et al.,(1,3); Beuth et al. (2)].

Basierend auf dem derzeit wissenschaftlichen Erkenntnisstand sind parenterale Arzneimittel, die wäßrige Mistelzubereitungen enthalten, entweder nicht auf einen bestimmten Mistellektingehalt eingestellt oder enthalten einen Mistellektingehalt von 50 ng/ml bis 350 ng/ml (EP 0 602 686 A2) oder gar darüber, z.B. Iscador Q 5 mg spezial mit 375 ng/ml.

Die wesentlichen Inhaltsstoffe von Mistelextrakten, die Mistellektine, sind Proteine. Als solche sind sie in wäßrigen Lösungen, welche die Grundlage für die Anwendung als parenterales Arzneimittel sind, nur kurzfristig stabil. Gelöste Proteine sind in vielfacher Weise der Möglichkeit chemischer und physikalischer Veränderungen ausgesetzt [Franks, F. (1993), (5,6); Wang, V-CJ., Hanson,MA. (1988), (7)]. Daher ist es unerläßlich, daß Proteine, die als Wirkstoffe von Arzneimitteln in wäßrigen Lösungen zum Einsatz kommen, stabilisiert werden.

Die EP 0 602 686 A2 beschreibt die Möglichkeit, wäßrige Mistellektin-Lösungen mit einem eingestellten Gehalt an Mistellektin zu stabilisieren. Mit den dort erwähnten Stabilisatoren gelingt es zwar, eine begrenzte Stabilität der Lösung bezüglich der Adsorption an Oberflächen zu gewährleisten, dies gelingt jedoch nur für einen Zeitraum, der zu kurz ist, um die Ansprüche für ein sicheres Arzneimittel zu erfüllen, wie sie z.B in den Arzneimittelprüfrichtlinien [Bundesanzeiger 1989; 41, 243 a, (8)] niedergelegt sind. So wird die Stabilität über eine ausreichende Laufzeit weder bei Lagerungstemperaturen von 25°C noch bei Kühlung (4-8 °C) erreicht (Abb. 1). Damit entsprechen nach EP 0 602 686 A2 hergestellte Arzneimittel nur für zu geringe Laufzeiten den allgemeinen Anforderungen zur Arzneimittelsicherheit, wo reproduzierbare Wirkstoffapplikation während der Laufzeit des Arzneimittels gefordert wird.

Der vorliegenden Erfindung liegt daher die weitere Aufgabe zugrunde, ein pharmazeutisches Mittel zur positiven Beeinflussung von immunologischen Parametern in der Tumortherapie oder bei Infektionskrankheiten bereitzustellen, das einen optimal wirksamen und sicher applizierbaren Gehalt an Wirkstoffen aus Mistelextrakten enthält, wobei die wirksamen Inhaltsstoffe reproduzierbar im Arzneimittel eingestellt und während der Laufzeit des Arzneimittels stabil sind.

Diese Aufgabe wird gelöst, mit einer Arzneimittelzubereitung gemäß Patentanspruch 1.

Erfindungsgemäß enthält die Zubereitung aus Mistelextrakten eine Lösung mit einem Gehalt von 5 bis 50 ng/ml, vorzugsweise 20-30 ng/ml an immunologisch aktivem, galaktosidbindendem Mistellektin in entsprechender Menge und sie erhäit durch Zugabe von Stabilisatoren im Konzentrationsbereich um 0,01 mg/ml die erforderliche Stabilität hinsichtlich Oberflächenbindung und Lagerstabilität.

Die Mistellektin-Zubereitungen können in einer anderen Ausführungsform auch als Lyophilisat bereitgestellt werden.

Überraschenderweise wurde erfindungsgemäß festgestellt, daß im Bereich deutlich geringerer Dosierungen von 15 bis 375 pg/kg Körpergewicht ein Wirkungsoptimum besteht, das insbesondere nach wiederholter Verabreichung über längere Zeit zutage tritt. Diese geringen Dosierungen verringern erheblich die üblichen Nebenwirkungen.

Die erfindungsgemäßen Arzneimittelzubereitungen sind auch gekennzeichnet durch eine Dosiseinheit von 15 bis 375 pg/kg Körpergewicht geeigneten Gehalt an biologisch aktivem Mistellektin.

Bevorzugt wird als Dosiseinheit ein analytisch kontrollierter Gehalt an galaktosidspezifischem Mistellektin für die Verabreichung von 25 bis 200 pg/kg Körpergewicht, insbesondere 75 pg/kg Körpergewicht.

Mit den erfindungsgemäßen Zubereitungen ist es erstmals möglich, reproduzierbar parenterale Arzneimittel herzustellen, die während der gesamten Laufzeit eine sichere Applikation zulassen, auch in Form handelsüblicher Einmalspritzen, wo sonst die Proteinadsorption an den lipophilen Kunststoffoberflächen sehr problematisch ist. Diese finden Verwendung zur Verbesserung von Immunparametern bei Menschen und Säugetieren, zur Tumortherapie sowie bei viralen Erkrankungen.

### Ermittlung des optimal wirksamen Mistellektingehalts

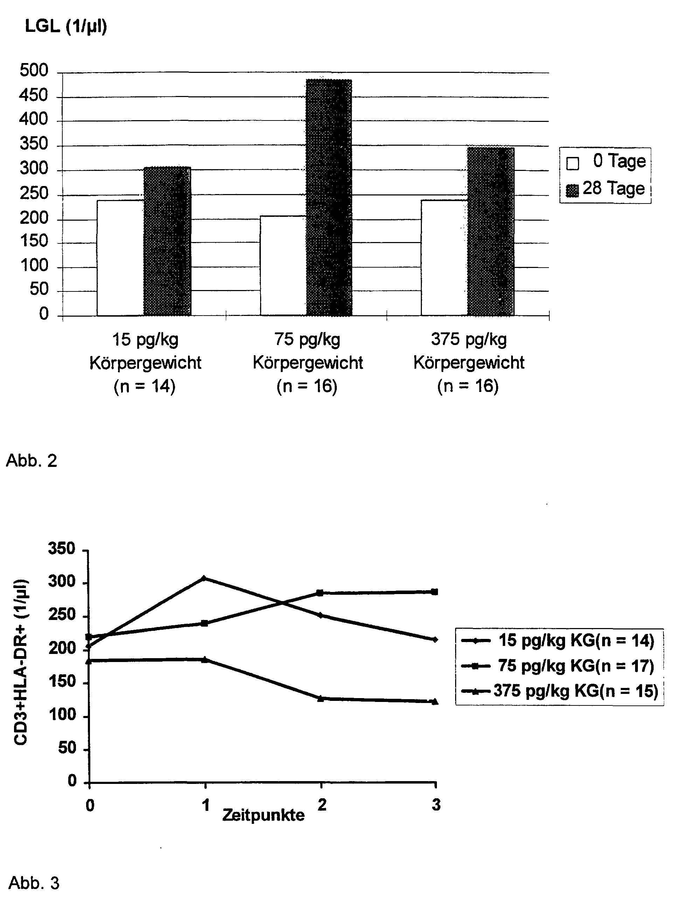

Für klinische Tests wurde ausgehend von einem wäßrigen Auszug aus unverholzten Mistelzweigen mit Blättern eine Injektionslösung mit 30 ng galaktosidbindendem Mistellektin pro ml - bestimmt als Mistellektin I - hergestellt. Die Anwendungszeit bei Krebspatienten betrug 4 Wochen bei zweimaliger Applikation pro Woche. Dabei wurde überraschend festgestellt, daß bereits bei einer Dosis von 75 pg/kg Körpergewicht die stärksten positiven Veränderungen von Immunparametern im Serum von Krebspatienten zu beobachten sind. Diese Befunde sind neu, denn nach dem Stand der Technik mußte man davon ausgehen, daß erst wesentlich höhere Dosierungen, und zwar 1 ng/kg Körpergewicht, eine positive Veränderung von Immunparametern hervorrufen. Vergleichende Tests ergaben, daß sowohl höhere als auch tiefere Dosierungen (15 pg/kg bzw. 375 pg/kg) geringere Veränderungen der Immunparameter CD 3+ HLA-DR + und LGL (large granular lymphocytes)-Zellzahl (Abb. 2 und Abb. 3) hervorrufen.

### Stabilisierung der Mistellektin-Zubereitung

Als Stabilisatoren für wäßrige Mistellektinzubereitungen werden Alkanole wie Polyole und Kohlenhydrate, hydrophile Polymere wie Polyvinylpyrrolidon, Polyvinylalkohol, nichtionische Tenside wie Polyalkylenglykole (Poloxamere), Polyoxyethylenfettsäureester (Polysorbate) und Proteine wie Albumin, speziell Humanserumalbumin, verwendet.

Stabilisierende Zusätze für Mistellektinpräparationen sind aus der EP 0 602 686 A2 bekannt, sie werden dort im Konzentrationsbereich von 0,1 mg/ml bis 10 mg/ml, vorzugsweise 5 mg/ml, der Lösung eingesetzt. Für die Analytik der erfindungsgemäßen Lösungen wird auf die EP 0602 686 A2 voll Bezug genommen.

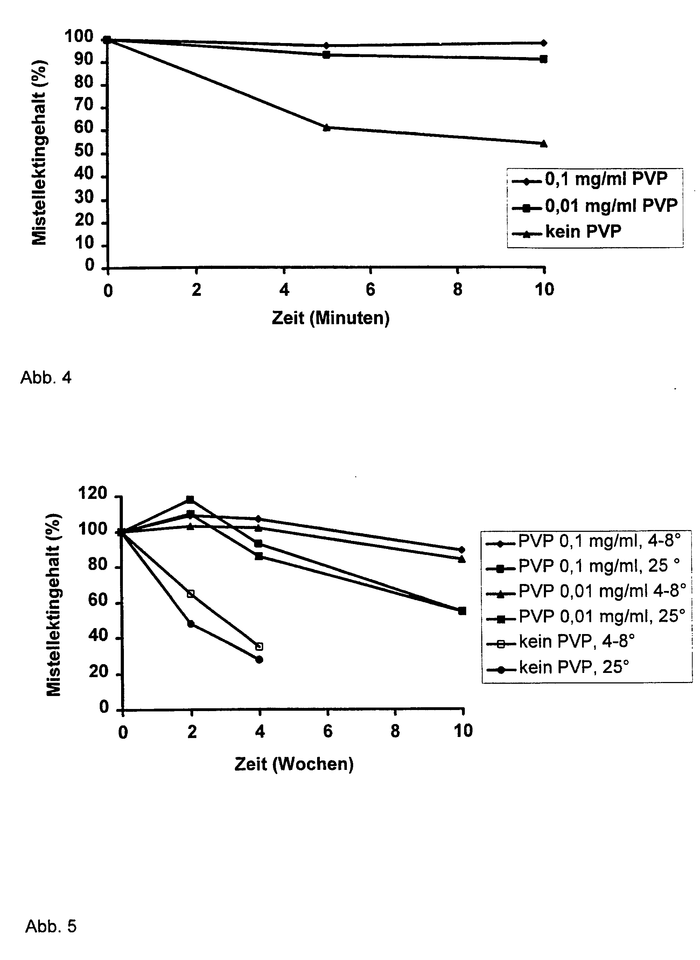

Überraschenderweise wurde nun gefunden, daß Konzentrationen von 0,01 mg/ml und geringer der genannten Stabilisatoren bezüglich der stabilisierenden Wirkung sich äquivalent verhalten. Dies läßt sich beispielhaft für die literaturbekannten Stabilisatoren Polyvidon (PVP) und Albumin zeigen. Diese Stabilisatoren erhöhen die Proteinstabilität hinsichtlich der Oberflächenbindung an lipophile Oberflächen (Abb. 4). Darüber hinaus wird auch für die Lagerstabilität eine vergleichbare Qualität erreicht, wenn geringere Konzentrationen der Hilfsstoffe (z.B. Polyvidon) zum Einsatz gelangen (Abb. 5).

Weiterhin wurde überraschend festgestellt, daß auch nichtionische Tenside wie Polyalkylenglykole (Blockpolymerisate aus Ethylenoxid und Propylenoxid, z.B. Pluronic) und Polyoxyethylenfettsäureester (z.B Tween 20) in denselben Konzentrationsbereichen ebenfalls stabilisierend wirken.

Mistelextraktzubereitungen werden als Dauertherapie angewendet. Die Reduzierung von Hilfsstoffen zur Stabilisierung trägt wesentlich zur Arzneimittelsicherheit bei, da z.B. Polymere wie Polyvidon und Polyalkylenglykole auf der Basis von Ethylen- und Propylenoxid nach parenteraler Applikation bei Nierenfunktionsstörungen verlangsamt ausgeschieden werden. Außerdem besteht die Gefahr, daß diese Stoffe akkumulieren. Diese Gefahr wird durch die Erniedrigung der Konzentration auf beispielsweise 0,01 mg/ml wesentlich reduziert.

In den erfindungsgemäßen Zubereitungen werden chelatbildende Zusätze wie Nitriloalkancarbonsäuren und deren Derivate eingesetzt, um die Lagerstabilität der Injektionslösungen zu erhöhen. Durch den Einsatz dieser Verbindungen, insbesondere als Alkalimetallsalze, zusätzlich zu den oben genannten Stabilisatoren, wird überraschenderweise eine drastisch verlängerte Stabilitätszeit für die Lagerung der Mistelextraktzubereitungen erzielt. Der Einfluß von chelatbildenden Zusätzen, die in parenteralen Arzneiformen bisher hauptsächlich zur Komplexierung von Metallen eingesetzt wurden (Wang, Hanson), auf Zubereitungen aus Mistelextrakten ist dem derzeitigen Stand der Technik nach unbekannt.

Geeignete Verbindungen sind NTA (Nitriloessigsäure und deren Salze), EDTA (Edetinsäure und deren Salze), CDTA (1,2-Diaminocyclohexan-N,N'-tetraessigsäure und deren Salze), N-(2-Hydroxyethyl)-Ethyldiamin-N,N,N'-triessigsäure und deren Salze, TTHA (Triethylentetraminhexaessigsäure und deren Salze). Es konnen eine oder mehrere der Einzelverbindungen zur Anwendung kommen.

Gemäß einer bevorzugten Ausführungsform wird als Nitriloalkancarbonsäurederivat Edetinsäure, insbesondere als Di-Natriumsalz eingesetzt. Vorzugsweise wird Di-Natriumedetat in einem Konzentrationsbereich von 0,001 mg/ml bis 0,1 mg/ml, insbesondere 0,01 mg/ml verwendet.

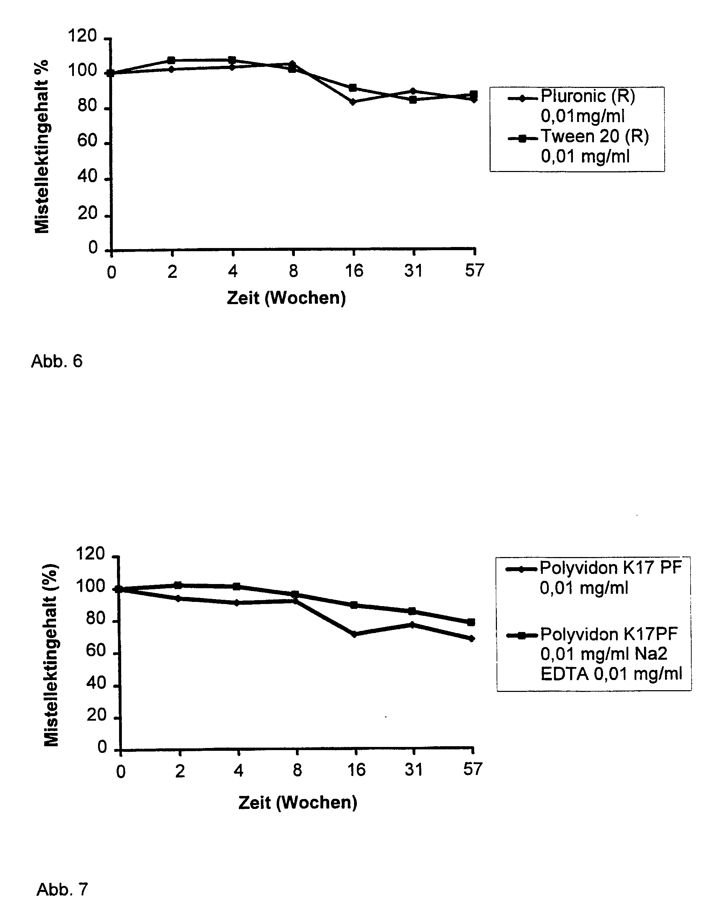

Die Überlegenheit der Kombination verschiedener Stabilisatorsubstanzen gegenüber einer Verbindung hinsichtlich der Lagerstabilität einer Mistelzubereitung wird anhand der Abnahme des Mistellektingehalts beispielhaft für Polyvidon bzw. Polyvidon mit Di-Natnumedetat deutlich (Abb. 7).

Der Zusatz von Stabilisatoren wie Polyvidon und Di-Natriumedetat beeinflußt die Aktivität von Mistelextraktzubereitungen nachweislich nicht. So weisen Mistelzubereitungen mit gleichem Mistellektingehalt sowohl mit Zusatz von PVP und Di-Natriumedetat wie auch ohne Zusatzstoffe die gleiche Aktivität im MOLT-4-Zytotoxizitätstest und bei der Freisetzung von Zytokinen von humanen Blutzellen auf. Die Zusatzstoffe selbst zeigen keine Aktivität in diesen Tests. Damit ist sichergestellt, daß die Zusatzstoffe die pharmakologische Wirkung der Mistelextraktzubereitungen nicht beeinflussen.

Die nachfolgenden Beispiele für Rezepturen stabiler Zubereitungen erläutern die Erfindung ohne sie zu beschränken.

### Beispiel 1: lnjektionslösung (5 l)

| | |
|---|---|
| wäßriger Auszug (1:1,1 - 1,5) aus unverholzten Mistelzweigen mit Blättern, entsprechend 30 ng/ml aktives Mistellektin | 0,2 - 0,7 g |
| Natriummonohydrogenphosphat -12-hydrat | 20,13 g |
| Natriumdihydrogenphosphat -2-hydrat | 1,88 g |
| Natriumchlorid | 37,50 g |
| Polyvidon K17PF | 0,05 g |
| Di-Natriumedetat | 0,05 g |
| Wasser | 4965 g |

### Beispiel 2 Injektionslösung (5 l)

| | |
|---|---|
| wäßriger Auszug (1:1,1 - 1,5) aus unverholzten Mistelzweigen mit Blättern entsprechend 30 ng/ml aktives Mistellektin | 0,2 - 0,7 g |
| Natriummonohydrogenphosphat -12-hydrat | 20,13 g |
| Natriumdihydrogenphosphat -2-hydrat | 1,88 g |
| Natriumchlorid | 37,50 g |
| Pluronic ® F68 | 0,05 g |
| Di-Natriumedetat | 0,05 g |
| Wasser | 4965 g |

### Beispiel 3 Injektionslösung (5 l)

| | |
|---|---|
| wäßriger Auszug (1:1,1 - 1,5) aus unverholzten Mistelzweigen mit Blättern entsprechend 30 ng/ml aktives Mistellektin | 0,2 - 0,7 g |
| Natriummonohydrogenphosphat -12-hydrat | 20,13 g |
| Natriumdihydrogenphosphat -2-hydrat | 1,88 g |
| Natriumchlorid | 37,50 g |
| Tween ® 20 | 0,05 g |
| Di-Natriumedetat | 0,05 g |
| Wasser | 4965 g |

### Kurze Beschreibung der Zeichnungen

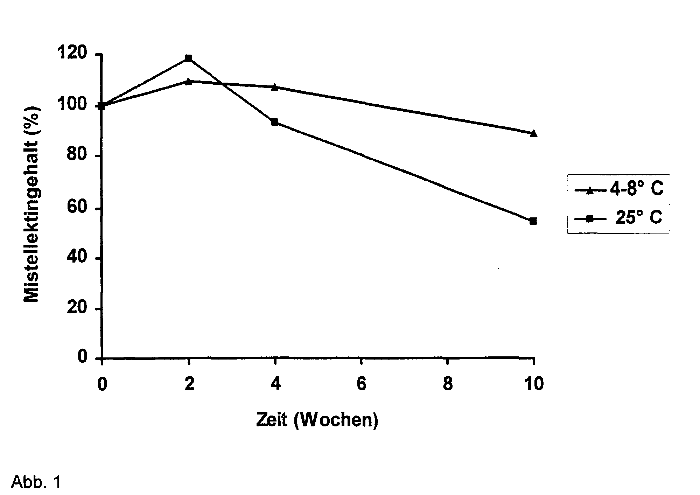

- Abb. 1: Lagerungsstabilität einer wäßrigen Zubereitung eines wäßrigen Auszugs (1:1,1-1,5) aus unverholzten Mistelzweigen mit Blättern eingestellt auf 65 ng/ml enthaltend 0,1 mg Polyvidon K17PF/ml, Mistellektinbestimmung mittels modifiziertem ELLA (9)
- Abb. 2: Medianwerte der LGL-Zellzahl während vierwöchiger Subkutanapplikation von PS76A2:
0 Tage: vor Applikationsbeginn, 28 Tage: Studienabschluß
- Abb. 3: Medianverläufe der aktivierten Lymphozyten (CD3+HLA-DR+) während vierwöchiger Subkutanapplikation von PS76A2:
0: vor Applikationsbeginn, 1: 24 Stunden nach Applikationsbeginn, 2: 48 Stunden nach Applikationsbeginn, 3: 28 Tage nach Applikationsbeginn
- Abb. 4: Abnahme des Mistellektingehalts in Prozent von Injektionslösungen enthaltend eine wäßrige Zubereitung eines wäßrigen Auszugs (1:1,1-1,5) aus unverholzten Mistelzweigen mit Blättern, eingestellt auf 65 ng/ml, in Abhängigkeit von der Verweildauer in Einmalspritzen (PE/PP) mit Gummikolben
- Abb. 5: Lagerungsstabilität einer Mistelzubereitung in 20 mM Phosphatpuffer pH 7,4 gemessen anhand des Mistellektingehalts in Prozent
- Abb. 6: Lagerungstabilität einer Mistelzubereitung in 20 mM Phosphatpuffer pH 7,4 gemessen anhand des Mistellektingehalts in Prozent, Lagerung bei 4-8° C
- Abb. 7: Lagerungsstabilität von Injektionslösungen enthaltend eine wäßrige Mistelzubereitung aus wäßrigem Auszug (1:1,1-1,5) aus unverholzten Mistelzweigen mit Blättern in 20 mM Phosphatpuffer, Lagerung bei 4-8° C

### Literatur

1. Hajto T, Hostanska K, Gabius H-J. Modulatory Potency of the β-Galactoside-specific Lectin from Mistletoe Extract (Iscador) on the Host Defense System in Vivo in Rabbits and Patients. Cancer Res. 1989; 49: 4803-4808.
2. Beuth, J, Ko H L, Tunggal L, Geisel J, Pulverer G. Vergleichende Untersuchungen zur immunaktiven Wirkung von Galaktosid-spezifischem Mistellektin: Arzneim. Forsch. 1993; 43: 166-169.
3. Hajto T, Hostanska K, Erfinder; Madaus AG, Anmelder. Lektinkonzentrate aus Mistelextrakten und entsprechende, stabilisierte Mistellektinpräparate, Verfahren zu ihrer Herstellung sowie diese enthaltende Arzneimittel und deren Verwendung zur Erhöhung der natürlichen Immunresistenz und/oder in der Tumor-Therapie. EP 0602 686 A 2. Offenlegung 22.06.1994.
4. Luther P, Becker H. Die Mistel. Berlin: Springer-Verlag 1987. 58-119.
5. Franks F. Conformational Stability of Proteins. In Franks F, editor. Protein Biotechnology. Totowa, New Jersey: Humana Press, 1993: 395-436.
6. Franks F. Storage Stabilization of Proteins. In Franks F, editor. Protein Biotechnology. Totowa, New Jersey: Humana Press, 1993: 489-532.
7. Wang Y-CJ, Hanson MA. Parenteral Formulations of Proteins and Peptides: Stability and Stabilizers. J. Parenter. Sci. Techn. 1988 suppl.; 42: S3-S26.
8. Allgemeine Verwaltungsvorschrift zur Anwendung der Arzneimittelprüfrichtlinien vom 14. Dezember 1989. Bundesanz. 1989; 41: 243 a.
9. Vang O, PiiLarsen K, Bog-Hansen TC. A New Quantitative and Highly Specific Assay for Lectinbinding Acitivity. In Bog-Hansen TC, van Driessche E, editors. Lectins Biology-Biochemistry-Clinical Biochemistry Vol. 5. Berlin: W. de Gruyter, 1986: 637-644.

## Patentansprüche

1. Arzneimittelzubereitung für die parenterale Verabreichung von Mistelextrakten als wäßriger Auszug aus Mistelzweigen, **dadurch gekennzeichnet, daß** sie als Lösung einen Gehalt von 5 - 50 ng/ml, vorzugsweise 20 bis 30 ng/ml, an immunologisch aktivem galaktosidbindenden Mistellektin enthält und einen Gehalt an Stabilisatoren um 0,01 mg/ml aufweist.

2. Arzneimittelzubereitung nach Anspruch 1, **dadurch gekennzeichnet, daß** sie Alkanole, insbesondere Polyole und Kohlenhydrate, hydrophile Polymere, insbesondere Polyvinylpyrrolidon, Polyvinylalkohol, nicht-ionische Tenside insbesondere Polyalkylenglykole und Polyoxyethylenfettsäureester und/oder Proteine, insbesondere Albumin, speziell Humanserumalbumin, als Stabilisatoren enthält.

3. Arzneimittelzubereitung nach einem der vorangehenden Ansprüche, **dadurch gekennzeichnet, daß** sie einen zusätzlichen Gehalt an Chelatbildnern von 0,1 -0,001 mg/ml, insbesondere von etwa 0,01 mg/ml aufweist.

4. Arzneimittelzubereitung nach Anspruch 3, **dadurch gekennzeichnet, daß** sie einen Gehalt an Alkalimetallsalz von Nitriloalkancarbonsäuren als Chelatbildner aufweist.

5. Arzneimittelzubereitung nach Anspruch 4, **dadurch gekennzeichnet, daß** als Nitriloalkancarbonsäurederivat Edetinsäure, vorzugsweise als Di-Natriumsalz, eingesetzt wird.

6. Arzneimittelzubereitung nach einem der vorangehenden Ansprüche in lyophilisierter Form.

7. Arzneimittelzubereitung nach einem der vorangehenden Ansprüche in Form einer Dosiseinheit, **dadurch gekennzeichnet, daß** sie einen Gehalt an biologisch aktivem galaktosidbindenden Mistellektin für die Applikation von 15 - 375 pg/kg Körpergewicht aufweist.

8. Arzneimittel nach Anspruch 7 in Form einer Dosiseinheit, **dadurch gekennzeichnet, daß** sie einen Lektingehalt für die Applikation von 25 - 200 pg/kg, insbesondere um 75 pg/kg Körpergewicht aufweist.

## Claims

1. Pharmaceutical preparation for the parenteral administration of mistletoe extracts as aqueous decoction from mistletoe twigs, **characterized in that** it comprises as solution a content of 5-50 ng/ml, preferably 20 to 30 ng/ml, of immunologically active galactoside-binding mistletoe lectin and has a content of stabilizers of around 0.01 mg/ml.

2. Pharmaceutical preparation according to Claim 1, **characterized in that** it comprises alkanols, especially polyols and carbohydrates, hydrophilic polymers, especially polyvinylpyrrolidone, polyvinyl alcohol, nonionic surfactants, especially polyalkylene glycols and polyoxyethylene fatty acid esters and/or proteins, especially albumin, specifically human serum albumin, as stabilizers.

3. Pharmaceutical preparation according to either of the preceding claims, **characterized in that** it has an additional content of chelating agents of 0.1-0.001 mg/ml, in particular of approximately 0.01 mg/ml.

4. Pharmaceutical preparation according to Claim 3, **characterized in that** it has a content of alkali metal salt of nitriloalkanecarboxylic acids as chelating agent.

5. Pharmaceutical preparation according to Claim 4, **characterized in that** edetic acid is employed as nitriloalkanecarboxylic acid derivative, preferably as disodium salt.

6. Pharmaceutical preparation according to any of the preceding claims, in lyophilized form.

7. Pharmaceutical preparation according to any of the preceding claims in the form of a dose unit, **characterized in that** it has a content of biologically active galactoside-binding mistletoe lectin for administration of 15-375 pg/kg of body weight.

8. Pharmaceutical according to Claim 7 in the form of a dose unit, **characterized in that** it has a lectin content for administration of 25-200 pg/kg, in particular around 75 pg/kg of body weight.

## Revendications

1. Préparation médicamenteuse pour l'administration parentérale d'extraits de gui sous forme d'extrait aqueux de rameaux de gui, **caractérisée en ce que**, sous forme de solution, elle a une teneur de 5 - 50 ng/ml, de préférence de 20 à 30 ng/ml, en mistellectine fixant le galactoside, immunologiquement active, et présente une teneur en stabilisants d'environ 0,01 mg/ml.

2. Préparation médicamenteuse selon la revendication 1, **caractérisée en ce qu'**elle contient en tant que stabilisants des alcools, en particulier des polyols, et des glucides, des polymères hydrophiles, en particulier de la polyvinylpyrrolidone, du poly(alcool vinylique), des tensioactifs non ioniques, en particulier des polyalkylèneglycols et des esters polyoxyéthyléniques d'acides gras et/ou des protéines, en particulier de l'albumine, notamment de l'albumine de sérum humain.

3. Préparation médicamenteuse selon l'une quelconque des revendications précédentes, **caractérisée en ce qu'**elle présente une teneur supplémentaire en agents chélateurs de 0,1 - 0,001 mg/ml, en particulier d'environ 0,01 mg/ml.

4. Préparation médicamenteuse selon la revendication 3, **caractérisée en ce qu'**elle présente une teneur en sel de métal alcalin d'acides nitriloalcanecarboxyliques en tant qu'agent chélateur.

5. Préparation médicamenteuse selon la revendication 4, **caractérisée en ce qu'**en tant que dérivé d'acide nitriloalcanecarboxylique on utilise l'acide édétique, en particulier sous forme de sel disodique.

6. Préparation médicamenteuse selon l'une quelconque des revendications précédentes, sous forme lyophilisée.

7. Préparation médicamenteuse selon l'une quelconque des revendications précédentes, sous forme d'une dose unitaire, **caractérisée en ce qu'**elle présente une teneur en mistellectine fixant le galactoside, immunologiquement active, pour l'administration de 15-375 pg/kg de poids corporel.

8. Médicament selon la revendication 7, sous forme d'une dose unitaire, **caractérisé en ce qu'**il présente une teneur en lectine pour l'administration de 25-200 pg/kg, en particulier d'environ 75 pg/kg de poids corporel.
